# EUROPEAN PATENT SPECIFICATION

(11) **EP 1 143 010 B1**
(45) Date of publication and mention of the grant of the patent: **20.12.2006**
(21) Application number: 00971442.9
(22) Date of filing: 13.10.2000
(51) Int. Cl.: C12Q 1/00, G01N 21/64, C12R 1/91

(54) **QUANTIFICATION OF PARALYZING TOXINS (PSP) BY FLUORIMETRY IN EXCITABLE CELLS**
QUANTIFIZIERUNG VON LÄHMENDEN TOXINEN (PSP) DURCH FLUORIMETRIE IN ERREGBAREN ZELLEN
QUANTIFICATION DE TOXINES PARALYSANTES (PSP) PAR FLUORIMETRIE DANS DES CELLULES EXCITABLES

(30) Priority: 15.10.1999 ES 9902278
(43) Date of publication of application: 10.10.2001
(73) Proprietor: UNIVERSIDADE DE SANTIAGO DE COMPOSTELA, 15706 Santiago de Compostela (ES)
(72) Inventor: Louzao Ojeda, Maria del C. Uni. de Santi. de Compo, 27002 Lugo (ES); Rodriguez Vieytes,Mercedes Uni. de Santi. de Compo, 27002 Lugo (ES); Botana Lopez, Luis Miguel Uni. de Santi. de Compo, 27002 Lugo (ES); Vieites Baptista de Sousa, Juan Manuel, Camp. Uni.36310 Vigo (ES); Leira Sanmartin, Francisco ANFACO - CECOPESCA, 36310 Vigo (ES)
(74) Representative: Davila Baz, Angel
(86) International application number: PCT/ES2000/000394
(87) International publication number: WO 2001/029250

(56) References cited:
- LAWRENCE J.F. ET AL.: 'Evaluation of a postcolumn electrochemical reactor for oxidation of paralytic shellfish poison toxins' J. AOAC INTERN. vol. 78, 1995, pages 698 - 704, XP002947880
- ALFONSO A. ET AL.: 'Comperative study of the stability of saxitoxin and neosaxitoxin in acidic solutions and lyophilized samples' TOXICON vol. 32, 1994, pages 1593 - 1598, XP002947881
- VIEYTES M.R. ET AL.: 'Solid-phase radioreceptor assay for paralytic shellfish toxins' ANALYTICAL BIOCHEMISTRY vol. 211, 1993, pages 87 - 93, XP002947882
- BURDASPAL P.A. ET AL.: 'Application of the fluorimetric method to the routine analysis of PSP in shellfish' ALIMENTARIA vol. 28, 1991, pages 23 - 27, XP002947883

## Description

Quantification of paralytic shellfish toxins (PSP) by fluorimetry in excitable cells. The fluorimetric technique uses a commercial membrane potential fluorescent dye to quantify paralyzing toxins in extracts of mussels contaminated during a red tide. Potential sensitive probes include carbocyanines, merocyanines and oxonols. In this case on use bisoxonol, a fluorescent probe included in the class oxonols. This dye distributes across the cellular membrane and allows measuring membrane potential changes in excitable cells. Those changes in membrane potential are shown as fluctuations in fluorescence of cells incubated with bisoxonol and they are recorded in a fluorimeter. In that way linear changes in fluorescence are related to changes in membrane potential of excitable cell. Quantification of paralyzing toxins by fluorimetry is based on the fact that PSP toxins inhibit cellular depolarization induced by veratridine (a toxin that activates sodium channels).

Bivalves, animals economically very important and abundant in our coasts, acquire, concentrate and accumulate toxins asociated to red tides with no important changes in its external morphology or vital functions. As mussels have no morphological changes there is a risk in public health, specially concerning with the intoxication with those contaminated mussels if they arrived to the market. All countries have programs to control recollection and commercialization of bivalves in contaminated areas. Paralyzing toxins (paralytic shellfish poison, PSP) are some of the most severe toxins belonging to marine toxins related to red tides. PSP received its name because ingestion of shellfish contaminated with those toxins may produce muscle paralysis or death in the most severe situation.

Chemically PSP toxins are derivatives of the imidazoline guanidinium group with different radical substitutive totaling up to 18 toxins. Structurally on be established 3 groups depending on the radical substitutive: carbamate toxins, N-sulfocarbamoyl toxins and decarbamoyl toxins. Accordingly to the number of charges in the molecule they can be classified in toxins of the saxitoxin (STX) group, toxins of the gonyautoxin (GTX) group and toxins of the C group. Absolute toxicity of PSP toxins varies enormously being the carbamate the most toxic.

The mechanism of action of PSP toxins is the competitive block of sodium channels in excitable cells, thus inhibiting the influx of sodium ions. Besides PSP there are other natural toxins with the same mechanism of action and causing dangerous intoxications. A clear example is tetrodotoxin that appears in puffer fish. There are also important some analogs of saxitoxin produced by freshwater algae.

The PSP detection method universally accepted is mouse bioassay (Sommer & Meyer: Paralytic shellfish poisoning. Arch. Path. 24: 560-598. 1937). However, limitations of this method, such as high variability in results obtained for the same extract of contaminated shellfish, low sensitivity and the high opposition to the sacrifice of animals, have allowed the research of alternative methods. Even though those alternative methods have multiple advantages like high sensitivity to low concentrations of toxins and less variability in results they have disadvantages too. Immunoassays (Chu and Fan: Indirect enzyme-linked immunosorbent assay for saxitoxin in shellfish. J Assoc Off Anal Chem 68, 13-16. 1985) detect a small group of toxins, which it exists the adequate antibody, being difficult the detection in the others. Even though radioreceptor methods are specific (Davio & Fontelo: A competitive displacement assay to detect saxitoxin and tetrodotoxin. Anal. Biochem. 141, 199-204 1984; Vieytes et al.: Solid-phase radioreceptor assay for paralytic shellfish toxins. Anal. Biochem. 210, 87-93. 1993) there is a tendency to displace them by no radioactive techniques. Cytotoxicity assays (Kogure y col. A tissue culture assay for tetrodotoxin, saxitoxin and related toxins. Toxicon 26, 191-197. 1988) increase sodium influx in neuroblastoma cells, then induce an increase in cellular volume and finally cellular death, originally were too slow and subjective. Later on those methods have improved in speed and objective detection by incorporating colorimetric techniques making simple the evaluation of results (Jellett, J. F. et al. Paralytic shellfish poison (saxitoxin family) bioassays: Automated endpoint determination and standarization on the in vitro tissue culture bioassay, and comparison with the standard mouse bioassay. Toxicon, 30, 1143-1156. 1992), but there are still lots of steps in the method requiring a few hours for toxin detection.

### SUMMARY OF THE INVENTION

Fluorimetric technique of detection and quantification of paralyzing toxins PSP, according to the invention is specific since it is based in the pharmacological mechanism of action of the toxins, it consists in the block of the sodium channels; it is sensitive and it is reliable for detecting levels of toxins lower than 0.2 µg/ml; and selective being accurate to detection of all PSP toxins. On quantify active toxins and has a high predictive value of toxicity of mollusks extracts.

The approach of this technique involves using the membrane potential fluorescent dye bis-oxonol that allows detection of changes in membrane potential induced by PSP toxins in excitable cells. Excitable cells employed belong to the human neuroblastoma cell line BE(2)-M17. Those cells express voltage dependent sodium channels and depolarize when they are incubated with toxins such as veratridine that binds to sodium channel, activate it and stimulate sodium entry inside the cell. PSP toxins by blocking sodium channels inhibit in a dose-dependent way depolarization induced by veratridine. This effect is the issue used as propose of the invention where on relate inhibition of depolarization (previously induced by veratridine), detected as a decrease in fluorescence, to PSP toxins concentration. Initially a standard relation between different percentages of depolarization inhibition and concentrations of saxitoxin is established. From this relation on quantify PSP toxins from the samples of contaminated mussels. Changes in membrane potential occur in a period of time of seconds this is different than others techniques that analyze the samples in 15 minutes.

### DESCRIPTION OF THE INVENTION

The technique can be separated 3 steps:
1) A suspension of cells from human neuroblastoma cell line BE(2)-M17 is kept at 37°C in a thermostated stirred quartz-microcuvette inside a spectrofluorometer where fluorescence is registered continuously at the λ excitation 540 nm and λ emission 560 nm. Membrane potential fluorescent dye bis-oxonol is added to a 2 nM final concentration and lasted for 5 minutes incubation before the next steps.
2) Addition of veratridine, a sodium channel-activating toxin that depolarizes the cell, which is recorded as an increase in fluorescence.
   3.1) Addition of different quantity of saxitoxin and consequently there is an inhibition dose dependent of depolarization of cells recorded as a decrease in fluorescence. With all of those data a standard relation between percentage of inhibition of depolarization and saxitoxin concentration is established.
   3.2) Addition of contaminated mollusks extracts and record of decrease of fluorescence as a consequence of the inhibition of cells depolarization due to PSP toxins. PSP toxins of the contaminated mollusks samples were quantified depending on the percentage of inhibition and the standard relation described before.

The invention is defined in the appended claims.

## Claims

1. A process for detecting and quantifying molluscs toxins which act by blocking sodium channels in the cells, **characterized in that** it comprises the following steps:
a) preparing a suspension of cells;
b) adding the membrane potential fluorescent dye bis oxonol;
c) adding the depolarizing toxin veratridine;
d) adding mollusc extracts;
e) recording the fluorescent intensity, for the purpose of detecting possibles decreases in membrane potential due to the presence of a paralytic shellfish poison in the mollusk; and
f) measuring the amount of toxin present in the mollusc extracts according to possible decreases detected and a standard on the basis of saxitoxins.

2. A process for detecting and quantifying toxins according to claim 1, **characterized in that** the excitable cells belong to the BE(2)-M17 human neuroblastoma cell line.

## Patentansprüche

1. Verfahren zum Nachweis und zur Quantifizierung von in Schalentieren vorhandenen Toxinen, die durch Blockierung der Natriumkanäle der Zellen wirksam sind, **gekennzeichnet durch** folgende Schritte:
a) Herstellung einer Zellsuspension;
b) Zugabe des membranpotentialabhängig fluoreszierenden Farbstoffs Bisoxonol;
c) Zugabe des depolarisierend wirkenden Toxins Veratridin;
d) Zugabe des jeweiligen Schalentierextrakts;
e) Messung der Fluoreszenzintensität, um eine mögliche Abnahme des Membranpotentials wegen eines eventuell im Schalentier vorhandenen paralytischen Schalentiergifts festzustellen; und
f) Messung der im Schalentierextrakt vorhandenen Toxinmenge anhand einer eventuell festgestellten Abnahme der Fluoreszenzintensität gegenüber einer Saxitoxine enthaltenden Vergleichsprobe.

2. Verfahren zum Nachweis und zur Quantifizierung von in Schaltieren vorhandenen Toxinen gemäß Anspruch 1, **dadurch gekennzeichnet, dass** die erregbaren Zellen zur Zelllinie humaner Blastome BE(2)-M17 gehören.

## Revendications

1. Procédé destiné à détecter et à quantifier des toxines de mollusques qui agissent en bloquant les canaux sodiques dans les cellules, **caractérisé en ce qu'**il comprend les étapes suivantes consistant à :
a) préparer une suspension de cellules ;
b) ajouter le colorant fluorescent dépendant du potentiel membranaire bis-oxonol ;
c) ajouter la toxine dépolarisante vératridine ;
d) ajouter des extraits de mollusque ;
e) enregistrer l'intensité de fluorescence afin de détecter des diminutions possibles du potentiel membranaire dues à la présence d'un poison paralytique de crustacé dans le mollusque ; et
f) mesurer la quantité de toxine présente dans les extraits de mollusque selon les diminutions possibles détectées et un standard fondé sur les saxitoxines.

2. Procédé destiné à détecter et à quantifier des toxines selon la revendication 1, **caractérisé en ce que** les cellules excitables appartiennent à la lignée de cellules de neuroblastome humain BE(2)-M17.
